Europäisches Patentamt

European Patent Office

Office européen des brevets

(19)

(11) Veröffentlichungsnummer: **0 356 965**
**A1**

## (12) EUROPÄISCHE PATENTANMELDUNG

(21) Anmeldenummer: 89115853.7

(22) Anmeldetag: 28.08.89

(51) Int. Cl.⁵: **C12N 9/64 , C12N 5/00**

(30) Priorität: 29.08.88 DE 3829244

(43) Veröffentlichungstag der Anmeldung:
**07.03.90 Patentblatt 90/10**

(84) Benannte Vertragsstaaten:
**AT BE CH DE ES FR GB GR IT LI LU NL SE**

(71) Anmelder: **BOEHRINGER MANNHEIM GmbH**
**Sandhofer Strasse 112-132**
**D-6800 Mannheim Waldhof(DE)**

(72) Erfinder: **Stockinger, Hubertus, Dr. rer. nat.**
**Unterholzstrasse 22**
**D-8122 Penzberg(DE)**
Erfinder: **Schetters, Hartmut, Dr. rer. nat.**
**Falkenstrasse 30**
**D-8122 Penzberg(DE)**
Erfinder: **Weidle, Ulrich, Dr. rer. nat.**
**Landwehrstrasse 56**
**D-8000 München 2(DE)**
Erfinder: **Debus, Elke, Dr. rer. nat.**
**Bräuhausstrasse 17**
**D-8132 Tutzing(DE)**

(74) Vertreter: **Huber, Bernhard, Dipl.-Chem. et al**
**Möhlstrasse 22 Postfach 860 820**
**D-8000 München 86(DE)**

(54) **Verfahren zur Herstellung von einkettigem t-PA.**

(57) Zur Herstellung von einkettigem t-PA unter Verwendung einer Kultur von eukaryontischen Zellen, die mit einem wenigstens ein Selektionsgen und das t-PA-Gen enthaltenden Vektor transfiziert sind und Gewinnung des t-PA aus dem Kulturmedium züchtet man die Zellen in einem Medium in völliger Abwesenheit von Plasmin.

EP 0 356 965 A1

## Verfahren zur Herstellung von einkettigem t-PA

Tissue Plasminogen Activator (t-PA) ist eine Serinprotease mit einem Molekulargewicht von 68.000 Dalton. Ihre einzige Funktion ist die Umwandlung von Plasminogen in Plasmin, ebenfalls einer Serinprotease mit einem breiten Wirkungsspektrum. Die Umwandlung geschieht durch die spezifische Spaltung der Peptidbindung von Arginin zu Valin an der Aminosäureposition 560 von Plasminogen. Wie andere Serinproteasen wird t-PA als eine einzige Polypeptidkette synthetisiert und dann durch Spaltung am unmittelbaren C-terminalen Ende an Position 257/258 in eine zweitkettige Form umgewandelt. Diese zwei Ketten mit einem Molekulargewicht von ca. 38.000 und 32.000 Dalton werden durch eine Disulfidbrücke zusammengehalten. Die leichtere Kette enthält den enzymatisch aktiven Bereich, während die schwerere Kette die sog. Finger- EGF- und Kringel-Domänen enthält. Mutante Formen von t-PA, bei denen die Spaltungsstelle Arg 257 eliminiert worden ist, erwiesen sich als enzymatisch inaktiv (Mol.Biol.Med. 3 (1986) 449-457). Während die leichte Kette die enzymatisch wichtige Rolle spielt, kommt der schweren Kette möglicherweise die durch Fibrin stimulierbare Funktion zu (J.Biol.Chem. 261 (1986) 3098-3102). Für eine therapeutische Anwendung ist es vorteilhaft, von Abbauprodukten freies, einkettiges t-PA ohne großen Aufwand herstellen zu können.

Beispielsweise ist aus EP 151996 A2 und Biochem.J. 226 (1985) 631-636 bekannt, rekombinanten einkettigen t-PA in eukaryonten Zellen in Gegenwart von Aprotinin herzustellen. Dabei kann nahezu vollständig einkettiger t-PA erhalten werden. Die Beimischung an zweikettigem t-PA ist so gering, daß die Reinheit für viele Anwendungsarten ausreicht.

Für eine therapeutische Anwendung ist jedoch zu fordern, daß t-PA ohne geringste Beimischungen von zweikettigem t-PA beschafft werden kann. Daher ist es Aufgabe der Erfindung, völlig von zweikettigem t-PA freien einkettigen t-PA zur Verfügung zu stellen.

Erfindungsgemäß wird diese Aufgabe gelöst durch ein Verfahren zur Herstellung von einkettigem t-PA unter Verwendung einer Kultur von eukaryontischen Zellen, die mit einem wenigstens ein Selektionsgen und das t-PA-Gen enthaltenden Vektor transfiziert sind und Gewinnung des t-PA aus dem Kulturmedium, das dadurch gekennzeichnet ist, daß man die Zellen in einem Medium in völliger Abwesenheit von Plasmin züchtet.

Als t-PA gelten im Rahmen der vorliegenden Erfindung nicht nur natürliches komplettes t-PA, sondern auch Derivate bzw. Muteine desselben, welche die typische biologische Eigenschaft desselben, also Umwandlung von Plasminogen in Plasmin, ebenfalls aufweisen. Eine Anzahl derartiger t-PA-Derivate ist dem Fachmann bekannt z.B. aus DE 36 43 158 A1 und EP 242 836 A1.

Die Erfindung beruht auf der überraschenden Feststellung, daß schon geringste Spuren von Plasmin bei der Züchtung dazu führen, daß eine gewisse Spaltung des einkettigen t-PA eintritt unter Bildung von zweikettigem t-PA. Für diese unerwünschte Spaltung reicht bereits die äußerst geringe Menge an Plasmin aus, welche an den zu züchtenden Zellen selbst anhaftet, wenn sie in einem serumhaltigen Medium vorkultiviert wurden. Daher wird vorzugsweise das Verfahren so durchgeführt, daß man die Zellen aus einem serumhaltigen Vorkulturmedium in ein serumfreies Zwischenkulturmedium überführt und darin während mindestens drei Generationen anzüchtet, diese Zwischenkultur unter Austausch des Zwischenkulturmediums gegen frisches serumfreies Zwischenkulturmedium mindestens einmal wiederholt, bis die Zellen plasminfrei sind, danach die Zellen in serumfreies Hauptkulturmedium überführt, nach Erreichen der stationären Wachstumsphase erntet und den einkettigen t-PA aus dem abgeernteten Kulturmedium gewinnt.

Als serumfreie Medien können im Rahmen der Erfindung alle Medien verwendet werden, die zur Züchtung von Eukaryonten geeignet sind. Geeignete handelsübliche serumfreie Medien, die dieser Forderung genügen, sind beispielsweise unter den Bezeichnungen RPMI 1640 (Morton, H.J. (1970) In Vitro 6, 89), DMEM (Dulbecco-modifiziertes Minimum Essential Medium, Goding, J.W. (1980) J.Immunol.Methods 39, 285) oder F12 (Ham R.G. (1965) PNAS USA 53, 288) bekannt. Bevorzugt verwendet man ein serumfreies Kulturmedium, welches ein Pentosan, ein Gemisch der natürlich vorkommenden L-Aminosäuren, Vitamine, Na, K, Mg, CA, ZN, Fe, Cu, Cl, $SO_4$, $NO_3$ und $PO_4$-Ionen, Pyruvat und eine Kohlenstoffquelle enthält. Besonders bevorzugt wird im Rahmen der Erfindung eine Mischung von DMEM- und F12-Medium etwa im Verhältnis 1:1 eingesetzt. Ganz besonders bevorzugt wird das in der Deutschen Patentanmeldung P 38 01 234 der gleichen Anmelderin beschriebene Medium. Dieses besteht im wesentlichen aus 2 bis 10 mg/l Transferrin, 2 bis 10 mg/l Insulin, 1 bis 10 g/l Pepton, 10 bis 500 mg/l ß-D-Xylopyranose, substituiert mit Phosphat-, Carboxy-und/oder Sulfatgruppen, 0,1 bis 0,5 mg/l Selenit und 0,1 bis 2 mg/l eines biologischen Polyamins. In diesem Medium weist die ß-D-Xylopyranose vorzugsweise ein Molekulargewicht von 1.000 bis 10.000 Dalton auf und besteht insbesondere aus einem entsprechenden Pentosanpolysulfat. Als Basismedium kann zweckmäßigerweise DMEM oder/und F12-Medium, wie oben schon beschrieben, insbesondere eine Mischung der beiden, verwendet werden. Anstelle von ß-D-Xylopyranose haben sich auch die entsprechenden ß-D-Ribo-, D-Arabino- und D-Lyxopyranose als gut geeignet erwiesen.

Zur Aufrechterhaltung der Stabilität der Plasmidtransfektion wird dem Medium zusätzlich ein dem Vektor entsprechendes Selektionsmittel hinzugefügt. Geeignete Selektionsmittel sind beispielsweise Neomycin, Hygromecin, Mycophenolsäure, Hypoxanthin, Xanthin, Aminopterin oder aber auch Metothrexat und Derivate. Die Vorkultur im serumfreien Medium erfolgt vorzugsweise unter mindestens zweimaligem Mediumwechsel, wobei jeweils mindestens drei Generationszeiten abgewartet werden.

Das erfindungsgemäß verwendete serumfreie Medium enthält kein Aprotinin. Es wurde gefunden, daß Aprotinin im Gegensatz zu den Lehren des Standes der Technik sich nachteilig auf die Stimulierbarkeit des gebildeten t-PA auswirkt.

Ein Mediumwechsel ist generell etwa alle vier Tage notwendig, um die Zellen mit den notwendigen Nährstoffen zu versorgen.

Bei der Hauptzellkultur werden die Zellen nach Erreichen der stationären Wachstumsphase geerntet und aus dem Überstand wird mit bekannten Methoden t-PA, wie z.B. in EMBO Journal 3 (1984) 51-56 beschrieben, isoliert. Zur Überprüfung auf Ein- und Zweikettigkeit wird so gewonnener t-PA zweckmäßig einer Gelanalyse unterzogen und durch eine Westernblotanalyse die Zustandsform nachgewiesen.

Als Zellen kommen im Rahmen der Erfindung alle in Kultur züchtbaren eukaryontischen Zellen in Betracht, welche t-PA produzieren können. Bevorzugte Beispiele hierfür sind CHO-Zellen (Chinese Hamster Ovary).

Die folgenden Beispiele erläutern die Erfindung weiter:

**Beispiel 1**

a) Transfektion eines das t-PA-Gen enthaltenden Vektors in CHO-Zellen

CHO dhfr⁻-Zellen (ECACC 88072103) werden, wie von R. Kaufmann und P. Sharp, J. Mol. Biol. 15 (1982) 601-621 mit dem Plasmid pBT 95 (DSM 3611P, DE-A 35 45 126) transfektiert.

b) Vorkultur:

1 x 10⁵ transfektierte Zellen werden pro ml eines handelsüblichen Mediums, welches 1 bis 20 % Serum enthält, ausgesät und 2 bis 6 Tage wachsen gelassen. Anschließend werden die Zellen abzentrifugiert, gewaschen und in einer Menge von 1 x 10⁵ Zellen/ml in einem serumfreien Medium resuspendiert, welches aus einer 1:1-Mischung von DMEM und F12-Medium be steht und zusätzlich 5 mg/l Transferrin, 5 mg/l Insulin, 1 mg/l Putrescin, 0,2 mg/l Selenit und 2 g/l Pepton aus Sojabohnen, sowie 100 mg/l Pentosanpolysulfat enthält. Nach 48 bis 96 Stunden werden die Zellen wieder geerntet, gewaschen und erneut in serumfreiem Medium gleicher Zusammensetzung kultiviert. Nach weiteren 72 Stunden werden die Zellen wiederum geerntet und gewaschen und dann durch Analyse überprüft und festgestellt, daß keinerlei Plasmin mehr nachweisbar ist und zu 100 % einkettiges t-PA vorliegt.

c) Hauptkultur:

1 x 10⁵ Zellen/ml werden in serumfreiem Medium der in der Vorkultur verwendeten Zusammensetzung ausgesät und bis zum Erreichen der maximalen Zelldichte in diesem Medium inkubiert. Anschließend werden die Zellen abgetrennt und aus dem Überstand wird t-PA nach dem Verfahren von Andreasen, EMBO Journal 3 (1984) 51-56 isoliert.

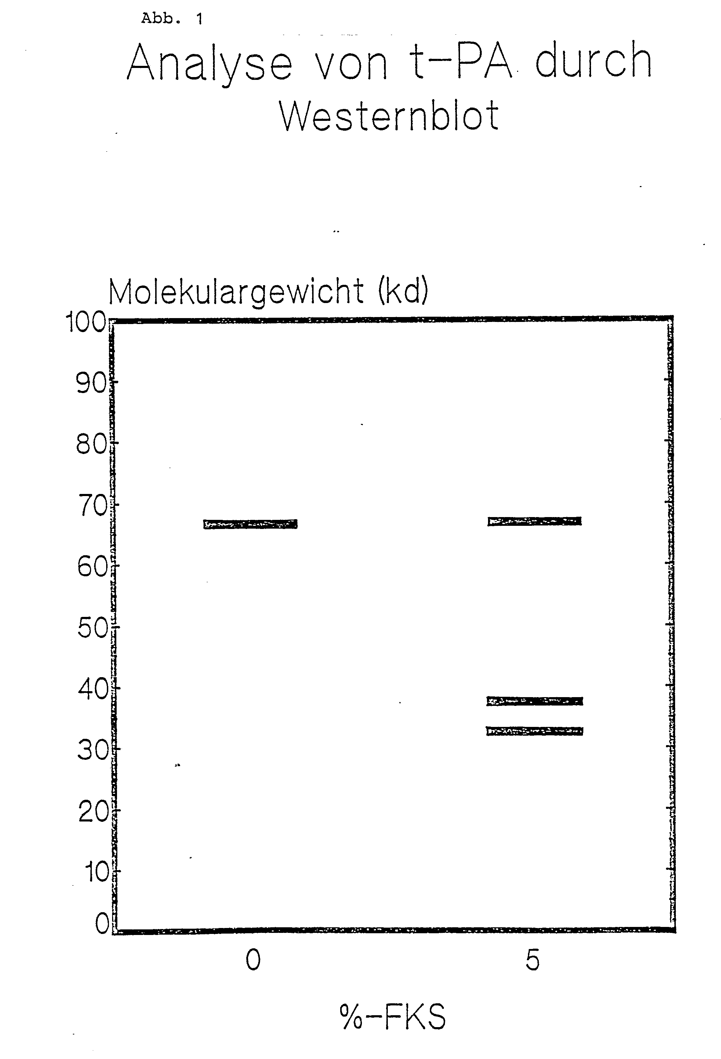

Die beigefügte Abbildung 1 zeigt eine Westernblotanalyse des so erhaltenen t-PA im Vergleich zu einem t-PA, welches in Gegenwart von 5 % fötalem Kälberserum bei sonst gleicher Zusammensetzung des Mediums erhalten wurde.

**Beispiel 2**

a) Transfektion von CHO-Zellen mit einem Vektor, der ein t-PA-Muteingen enthält

Plasmid pePa 144 (hergestellt nach Beispiel 2 von EP-A 0 242 836) wird, wie in Beispiel 1a) beschrieben, in CHO-Zellen eingeführt.

Die Kultur der Zellen erfolgt wie in Beispiel 1b) und c) beschrieben. Es wird ebenfalls 100 % einkettiges t-PA-Mutein erhalten.

**Beispiel 3**

a) Transfektion von CHO dhfr⁻-zellen mit einem Vektor, der ein t-PA-Muteingen enthält.

Als t-PA-Muteine wurden verwendet ΔK2 und Δ(K2 + EGF) (vgl. DE 38 25 253).

CHO dhfr⁻-Zellen, ECACC 88072103, wurden wie von Urlaub und Chasin, Proc. Natl. Acad. Soc. USA 77 (1980), 4216-4220 beschrieben, gezüchtet und vermehrt. Zur DNA-Transfektion wurden Calciumphosphat-Präzipitate mit 20 μg pSV (ΔK2)-dhfr (DSM 4721) und pSVΔ(K2EGF)-dhfr (DSM 4720), wie von Graham and Van der Eb, Virology 52 (1973), 456467 beschrieben, in einem Volumen von 4 ml hergestellt. 1 ml des Präzipitats wurde zu 3x10⁵ bis 1x10⁶ Zellen in 10 ml Medium in 9 cm Kulturschalen zugegeben. Die Zellen wurden 8 bis 16 Stunden mit dem Präzipitat inkubiert, dann das Medium entfernt und die Zellen mit 10 ml TBS (25 mmol Tris-HCl, pH 7,4, 137 mmol NaCl, 5 mmol KCl, 0,6 mmol $Na_2HPO_4$) gewaschen.

Die Kultivierung der Zellen erfolgt wie in Beispiel 1b) und c) beschrieben. Es werden ebenfalls 100 % einkettige t-PA-Muteine erhalten.

## Beispiel 4

pBT95 wird, wie in Beispiel 1a) beschrieben, in die CHO-Zellen (ATCC CCL 61, CHO K1) eingebracht und, wie in Beispiel 1b) und c) beschrieben, kultiviert. Auch in diesen Zellinien wird 100 % einkettiges t-PA erhalten.

## Ansprüche

1. Verfahren zur Herstellung von einkettigem t-PA unter Verwendung einer Kultur von eukaryontischen Zellen, die mit einem wenigstens ein Selektionsgen und das t-PA-Gen enthaltenden Vektor transfiziert sind und Gewinnung des t-PA aus dem Kulturmedium,

**dadurch gekennzeichnet,**

daß man die Zellen in einem Medium in völliger Abwesenheit von Plasmin züchtet.

2. Verfahren nach Anspruch 1,

**dadurch gekennzeichnet,**

daß man die Zellen aus einem serumhaltigen Vorkulturmedium in ein serumfreies Zwischenkulturmedium überführt und darin während mindestens drei Generationen anzüchtet, diese Zwischenkultur unter Austausch des Zwischenkulturmediums gegen frisches serumfreies Zwischenkulturmedium mindestens einmal wiederholt, bis die Zellen plasminfrei sind, danach die Zellen in serumfreies Hauptkulturmedium überführt, nach Erreichen der stationären Wachstumsphase erntet und den einkettigen t-PA aus dem abgeernteten Kulturmedium gewinnt.

3. Verfahren nach Anspruch 1 oder 2,

**dadurch gekennzeichnet,**

daß man ein serumfreies Kulturmedium verwendet, welches ein Pentosan, ein Gemisch der natürlich vorkommenden L-Aminosäuren, Vitamine, Na, K, Mg, CA, ZN, Fe, Cu, Cl, $SO_4$, $NO_3$ und $PO_4$-Ionen, Pyruvat und eine Kohlenstoffquelle enthält.

4. Verfahren nach einem der vorhergehenden Ansprüche,

**dadurch gekennzeichnet,**

daß man die Zwischenkulturen und die Hauptkultur in Abwesenheit von Aprotinin durchführt.

Abb. 1

# Analyse von t-PA durch Westernblot

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 248 675 (GENENTECH INC.) <br> * Ansprüche; Seite 3, Zeile 18-62; Seite 5, Zeilen 25-46 * <br> --- | 1-4 | C 12 N 9/64 <br> C 12 N 5/00 |
| Y | EP-A-0 113 319 (E.L. WILSON) <br> * Ansprüche; Seite 15, Zeilen 1-8 * <br> --- | 1-4 | |
| Y | EP-A-0 112 122 (SOUTH AFRICAN INVENTIONS DEV.) <br> * Seite 8, Zeilen 6-21; Ansprüche * <br> --- | 1-4 | |
| D,Y | BIOCHEM. J., Band 226, 1985, Seiten 631-636, GB; E.K.O. KRUITHOF et al.: "Humun tissue-type plasminogen activator. Production in continuous serum-free cell culture and rapid purification" <br> * Insgesamt * <br> --- | 1-4 | |
| D,Y <br> P | DE-A-3 801 236 (BOEHRINGER) <br> * Insgesamt * <br> ------ | 1-4 | RECHERCHIERTE SACHGEBIETE (Int. Cl.5) <br><br> C 12 N |

Der vorliegende Recherchenbericht wurde für alle Patentansprüche erstellt

| Recherchenort | Abschlußdatum der Recherche | Prüfer |
|---|---|---|
| DEN HAAG | 10-11-1989 | HUBER-MACK A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

...........................................................................................
& : Mitglied der gleichen Patentfamilie, übereinstimmendes Dokument